# EUROPÄISCHE PATENTANMELDUNG

(11) **EP 2 338 460 A1**
(43) Veröffentlichungstag der Anmeldung: **29.06.2011**
(21) Anmeldenummer: 10174571.9
(22) Anmeldetag: 31.08.2010
(51) Int. Cl.: A61K 8/02, A61Q 5/04, A61K 8/04

(54) **Dauerwellsystem**

(30) Priorität: 03.09.2009 DE 102009029183
(71) Anmelder: Henkel AG & Co. KGaA, 40589 Düsseldorf (DE)
(72) Erfinder: Emmerling, Winfried, 25436, Tornesch (DE); Rautenberg-Groth, Birgit, 25479, Ellerau (DE)

(57) **Zusammenfassung**

Ein Dauerwellsystem in Form eines "Kit-of-parts", umfassend (i) mindestens einen mit einer Verschäumvorrichtung ausgestatteten ersten nichtaerosol-Behälter, der ein flüssiges Wellmittel, enthaltend in einem kosmetischen Träger eine Kombination mindestens einer keratinreduzierenden Verbindung mit mindestens einem Tensid, umfasst und (ii) mindestens einen mit einer Verschäumvorrichtung ausgestatteten zweiten nichtaerosol-Behälter, der ein flüssiges Fixiermittel, enthaltend in einem kosmetischen Träger eine Kombination aus mindestens einem Oxidationsmittel mit mindestens einem Tensid, umfasst, kann insbesondere in der Selbstanwendung leicht gehandhabt werden. Es werden gute Schaumparameter des resultierenden Nichtaerosolschaums und gute Wellergebnisse erzielt.

## Beschreibung

Die Erfindung betrifft ein Dauerwellsystem in Form eines "Kit-of-parts", umfassend (i) mindestens einen mit einer Verschäumvorrichtung ausgestatteten ersten nichtaerosol-Behälter, der ein flüssiges Wellmittel, enthaltend in einem kosmetischen Träger eine Kombination mindestens einer keratinreduzierenden Verbindung mit mindestens einem Tensid, umfasst und (ii) mindestens einen mit einer Verschäumvorrichtung ausgestatteten zweiten nichtaerosol-Behälter, der ein flüssiges Fixiermittel, enthaltend in einem kosmetischen Träger eine Kombination aus mindestens einem Oxidationsmittel mit mindestens einem Tensid, umfasst. Ferner sind die Verwendung dieses Kits im Rahmen einer dauerhaften Umformung keratinhaltiger Fasern sowie ein entsprechendes Anwendungsverfahren Gegenstand der Erfindung.

Die dauerhafte Verformung von Keratinfasern wird üblicherweise so durchgeführt, daß man die Faser mechanisch verformt und die Verformung durch geeignete Hilfsmittel festlegt. Vor und/oder nach dieser Verformung behandelt man die Faser mit der wäßrigen Zubereitung einer keratinreduzierenden Verbindung und spült nach einer Einwirkungszeit mit Wasser oder einer wäßrigen Lösung. In einem zweiten Schritt behandelt man dann die Faser mit der wäßrigen Zubereitung eines Oxidationsmittels. Nach einer Einwirkungszeit wird auch dieses ausgespült und die Faser von den mechanischen Verformungshilfsmitteln (Wickler, Papilloten) befreit.

Die wäßrige Zubereitung der keratinreduzierenden Verbindung ist üblicherweise alkalisch eingestellt, damit zum einen genügender Anteil der Thiolfunktionen deprotoniert vorliegt und zum anderen die Faser quillt und auf diese Weise ein tiefes Eindringen der keratinreduzierenden Verbindung in die Faser ermöglicht wird. Die keratinreduzierende Verbindung spaltet einen Teil der Disulfid-Bindungen des Keratins zu -SH-Gruppen, so daß es zu einer Lockerung der Peptidvernetzung und infolge der Spannung der Faser durch die mechanische Verformung zu einer Neuorientierung des Keratingefüges kommt. Unter dem Einfluß des Oxidationsmittels werden erneut Disulfid-Bindungen geknüpft, und auf diese Weise wird das Keratingefüge in der vorgegebenen Verformung neu fixiert. Ein bekanntes derartiges Verfahren stellt die Dauerwell-Behandlung menschlicher Haare dar. Dieses kann sowohl zur Erzeugung von Locken und Wellen in glattem Haar als auch zur Glättung von gekräuselten Haaren angewendet werden.

Eine negative Begleiterscheinung der so durchgeführten Dauerwellung des Haares ist ein Verspritzen bzw. Herabtropfen der Well- und der Fixierflüssigkeit während des Auftragens und während der Einwirkzeit. Dies gilt verstärkt im Rahmen einer Selbstanwendung.

Es hat in der Vergangenheit nicht an Versuchen gefehlt, Dauerwellformulierungen zu entwickeln, welche diese Nachteile nicht aufweisen.

Es bestand somit die Aufgabe, ein System für die dauerhafte Verformung von keratinhaltigen Fasern bereitzustellen, bei dessen Anwendung das Herabrinnen bzw. Herabtropfen der kosmetischen Zusammensetzungen von der Faser reduziert oder ganz ausgeschlossen wird. Der Verbraucher soll zusätzlich ein Dauerwellsystem an die Hand bekommen, welches einfach und sicher in der Handhabung ist und eine Selbstanwendung erleichtert.

Überraschenderweise wurde gefunden, dass folgendes Dauerwellsystem diese Aufgabe in erstaunlich gutem Maße löst. Es stellt leicht zu applizierende Wellmittel bzw. Fixiermittel zur Verfügung, welche in Form eines hinreichend stabilen Nichtaerosolschaums auf das Haar aufgebracht werden. Es hat sich gezeigt, dass sich das Wellmittel bzw. Fixiermittel in Form eines Nichtaerosolschaums nicht nur hervorragend im Haar verteilen lässt sondern ebenso aufs Beste in die Haarfaser eindringt und dort seine Wirkung entfaltet.

Ein erster Gegenstand der Erfindung ist ein Dauerwellsystem in Form eines "Kit-of-parts", umfassend
(i) mindestens einen mit einer Verschäumvorrichtung ausgestatteten ersten nichtaerosol-Behälter, der ein flüssiges Wellmittel enthält, welches in einem kosmetischen Träger eine Kombination mindestens einer keratinreduzierenden Verbindung mit mindestens einem Tensid umfasst, und
(ii) mindestens einen mit einer Verschäumvorrichtung ausgestatteten zweiten nichtaerosol-Behälter, der ein flüssiges Fixiermittel enthält, welches in einem kosmetischen Träger eine Kombination aus mindestens einem Oxidationsmittel mit mindestens einem Tensid umfasst.

Erfindungsgemäß werden Druckgasbehälter, mit deren Hilfe ein Produkt durch den erhöhten Gasinnendruck des Behälters über Betätigung eines Ventils ausgebracht wird als "Aerosole" bezeichnet (siehe K. Schrader, *Grundlagen und Rezepturen der Kosmetika,* Dr. Alfred Hüthig Verlag, Heidelberg, 1989.). Als "Nichtaerosol" wird im Umkehrschluss zur Aerosoldefinition ein Behälter unter Normaldruck (d.h. Aussendruck = Innendruck) definiert, mit dessen Hilfe ein Produkt mittels mechanischer Einwirkung, z.B. durch ein Pump- oder Quetschsystem ausgebracht wird.

Eine Verschäumvorrichtung ist erfindungsgemäß eine technische Vorrichtung, mit deren Hilfe eine flüssige Zusammensetzung (hier Wellmittel beziehungsweise Fixiermittel) in eine schaumförmige Zusammensetzung umgewandelt wird.

Als "flüssige Zusammensetzung" gilt erfindungsgemäß eine Zusammensetzung, die bei 1013 mbar und 20°C flüssig ist.

Es ist erfindungsgemäß bevorzugt, wenn der erste nichtaerosol-Behälter des Wellmittels und der zweite nichtaerosol-Behälter des Fixiermittels als Quetschbehälter ausgestaltet sind.

Ein nichtaerosol-Behälter in Form eines Quetschbehälters umfasst üblicherweise einen Quetsch-Vorratsbehälter, der das Wellmittel bzw. das Fixiermittel enthält und dessen elastische Wandungen sich reversibel zusammendrücken lassen. Der Quetsch-Vorratsbehälter weist am oberen Ende eine Öffnung auf, die in eine Verschäumvorrichtung mündet. Der Quetsch-Vorratsbehälter besitzt bevorzugt ein inneres Volumen von 100 bis 200 mL. Der Quetsch-Vorratsbehälter wird bevorzugt derart mit dem Wellmittel bzw. Fixiermittel befüllt, so dass mindestens 20 % des Innenvolumens, besonders bevorzugt mindestens 30 % des Innenvolumens mit Luft gefüllt ist.

Bevorzugt werden die Wandungen des Quetsch-Vorratsbehälters aus einem Polyolefin, wie beispielsweise Polypropylen (PP), high density Polyethylen (HDPE), medium density Polyethylen (MDPE), low density Polyethylen (LDPE), linear low density Polyethylen (LLDPE), gefertigt. Darunter ist Polypropylen (PP) bevorzugt geeignet.

Die Verschäumvorrichtung umfasst bevorzugt eine Mischkammer, mindestens eine Schaumerzeugungseinrichtung und einen Schaumkanal. Letzterer ist gegebenenfalls unterteilt in einen Schaumführungskanal und Schaumaustrittskanal. Die Öffnung des Quetsch-Vorratsbehälters grenzt üblicherweise an die Mischkammer. Die Schaumerzeugungseinrichtung befindet sich üblicherweise zwischen Mischkammer und Schaumkanal.

Die Mischkammer weist üblicherweise einen Einlass für die flüssige Zusammensetzung aus dem Quetsch-Vorratsbehälter und einen Einlass für Luft aus dem Quetsch-Vorratsbehälter auf. Die Zuführung der flüssigen Zusammensetzung (i.e. Wellmittel bzw. Fixiermittel) zum Einlass in die Mischkammer erfolgt bevorzugt durch ein Steigrohr, welches mit einem Ende am Einlass der Mischkammer befestigt ist und mit seinem anderen Ende in die flüssige Zusammensetzung eintaucht.

Die Schaumerzeugungseinrichtung ist bevorzugt derart in der Verschäumvorrichtung angeordnet, dass bei Durchführung eines Pumpvorganges am Pumpbehälter oder Quetschvorganges am Quetschvorratsbehälter Luft und das flüssige Wellmittel (bzw. Luft und das flüssige Fixiermittel) gleichzeitig in die Mischkammer gelangen und von dort aus durch die Schaumerzeugungseinrichtung hindurch in den Schaumkanal aufschäumen. Die Schaumerzeugungseinrichtung umfasst dabei bevorzugt mindestens ein poröses Material, wie z.B. eine poröse Keramik, oder besonders bevorzugt mindestens ein Netz. Liegt die Schaumerzeugungseinrichtung als Netz vor, so weist das Netz bevorzugt eine Lochdichte von 50 bis 220 mesh (mesh = Anzahl der Löcher pro Inch), besonders bevorzugt von 90 bis 200 mesh, ganz besonders bevorzugt von 125 bis 175 mesh auf.

Die Verschäumvorrichtung umfasst bevorzugt eine Mischkammer, mindestens eine Schaumerzeugungseinrichtung in Form eines Netzes und einen Schaumkanal. Letzterer ist gegebenenfalls unterteilt in einen Schaumführungskanal und Schaumaustrittskanal.

Im Rahmen einer besonders bevorzugten Ausführungsform wird bei Durchführung eines Pump- oder Quetschvorganges Luft und das flüssige Wellmittel (bzw. Luft und das flüssige Fixiermittel) gleichzeitig in die Mischkammer befördert und von dort aus durch eine erste Schaumerzeugungseinrichtung in Form eines Netzes gedrückt, wobei der entstandene Schaum in den Schaumführungskanal gelangt, wo er vor Austritt aus dem nichtaerosol Behälter durch eine zweite Schaumerzeugungseinrichtung in Form eines Netzes in den Schaumaustrittskanal gedrückt wird. Das erste Netz weist bevorzugt eine Lochdichte von 50 bis 220 mesh (mesh = Anzahl der Löcher pro Inch), besonders bevorzugt von 90 bis 200 mesh, ganz besonders bevorzugt von 125 bis 175 mesh auf. Das zweite Netz weist bevorzugt eine Lochdichte von 160 bis 280 mesh, besonders bevorzugt von 175 bis 245 mesh und ganz besonders bevorzugt von 180 bis 220 mesh, auf.

Die in dem Wellmittel enthaltenen keratinreduzierenden Verbindungen werden bevorzugt ausgewählt unter Verbindungen mit mindestens einer Thiolgruppe sowie deren Derivate, aus Sulfiten, Hydrogensulfiten, Disulfiten.

Verbindungen mit mindestens einer Thiolgruppe sowie deren Derivate sind beispielsweise Thioglykolsäure, Thiomilchsäure, Thioäpfelsäure, Phenylthioglykolsäure, Mercaptoethansulfonsäure sowie deren Salze und Ester (wie z.B. Isooctylthioglycolat und Isopropylthioglycolat), Cysteamin, Cystein, Bunte Salze und Salze der schwefligen Säure. Bevorzugt geeignet sind die Monoethanolammoniumsalze oder Ammoniumsalze der Thioglykolsäure und/oder der Thiomilchsäure sowie die freien Säuren. Diese werden in dem Wellmittel bevorzugt in Konzentrationen von 0,5 bis 2,0 mol/kg bei einem pH-Wert von 5 bis 12, insbesondere von 7 bis 9,5, eingesetzt. Zur Einstellung dieses pH-Wertes enthalten die Wellmittel üblicherweise Alkalisierungsmittel wie Ammoniak, Alkali- und Ammonium-carbonate und -hydrogencarbonate oder organische Amine wie Monoethanolamin.

Beispiele für keratinreduzierende Verbindungen der Disulfite, die in dem Wellmittel enthaltenen sein können, sind Alkalidisulfite, wie z.B. Natriumdisulfit (Na₂S₂O₅) und Kaliumdisulfit (K₂S₂O₅), sowie Magnesiumdisulfit und Ammoniumdisulfit ((NH₄)₂S₂O₅). Ammoniumdisulfit kann dabei erfindungsgemäß bevorzugt sein. Beispiele für keratinreduzierende Verbindungen der Hydrogensulfite, die dem Wellmittel enthaltenen sein können, sind Hydrogensulfite als Alkali-, Magnesium-, Ammonium- oder Alkanolammonium-Salz auf Basis eines C₂-C₄-Mono-, Di- oder Trialkanolamins. Ammoniumhydrogensulfit kann dabei ein besonders bevorzugtes Hydrogensulfit sein. Beispiele für keratinreduzierende Verbindungen der Sulfite, die dem Wellmittel enthalten sein können, sind Sulfite als Alkali-, Ammonium- oder Alkanolammonium-Salz auf Basis eines C₂-C₄-Mono-, Di- oder Trialkanolamins. Ammoniumsulfit ist dabei bevorzugt. Der pH-Wert des Wellmittels wird bei Verwendung von Sulfit und/oder Disulfit und/oder Hydrogensulft bevorzugt auf einen Wert im Neutralbereich von pH 5 bis 8, bevorzugt von pH 6 bis 7,5 eingestellt.

Bevorzugte schwefelhaltige, keratinreduzierende Verbindungen sind Cysteamin, Cystein, Bunte Salze und Salze der schwefligen Säure, Thioglykolsäure, Thiomilchsäure, Thioäpfelsäure, Mercaptoethansulfonsäure sowie den Salzen und Estern der vorgenannten Thiosäuren.

Die keratinreduzierende Verbindung wird bevorzugt in einer Menge von 5 bis 20 Gew.-%, bezogen auf das gesamte Wellmittel, eingesetzt. Der pH-Wert des Wellmittels liegt dabei wiederum bevorzugt im Bereich von pH 5 bis 12, insbesondere von pH 7 bis 9,5.

Das flüssige Fixiermittel enthält als Oxidationsmittel bevorzugt Wasserstoffperoxid und gegebenenfalls die zur Stabilisierung wässriger Wasserstoffperoxidzubereitungen üblichen Stabilisatoren. Der pH-Wert solcher wässriger H₂O₂-Zubereitungen, die etwa 0,5 bis 3,0 Gew.-% H₂O₂ enthalten, liegt bevorzugt bei 2 bis 4; er wird durch anorganische Säuren, bevorzugt Phosphorsäure eingestellt. Weitere mögliche Oxidationsmittel sind Natrium- und/oder Kaliumbromat. Solche Bromate werden in Konzentrationen von 1 bis 10 Gew.-% eingesetzt und der pH-Wert der Lösungen wird auf 4 bis 7 eingestellt.

Bevorzugte Tenside für das Wellmittel und/oder Fixiermittel werden unabhängig voneinander ausgewählt unter anionischen Tensiden, zwitterionischen Tensiden, amphoteren Tenisden, nichtionischen Tensiden, kationischen Tensiden.

Es hat sich als besonders bevorzugt erwiesen, wenn die erfindungsgemäßen Mittel zusätzlich mindestens ein nichtionisches Tensid enthalten. Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 100 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht.

Als ganz besonders bevorzugte nichtionische Tenside haben sich Aminoxide, sowie Anlagerungsprodukte von 2 bis 100 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, erwiesen. Zubereitungen mit hervorragenden Eigenschaften werden ebenfalls erhalten, wenn sie als nichtionische Tenside C₁₂-C₃₀-Fettsäuremono- und -diester von Anlagerungsprodukten von 1 bis 30 Mol Ethylenoxid an Glycerin und/oder Anlagerungsprodukte von 5 bis 60 Mol Ethylenoxid an Rizinusöl und gehärtetes Rizinusöl enthalten.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂CH₂O)ₓ-OSO₃H , in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906 beschrieben sind,
- Amidethercarbonsäuren,
- Kondensationsprodukte aus C₈-C₃₀-Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate und Ethercarbonsäuren mit 10 bis 18 C-Atomen in der Alkylgruppe und bis zu 12 Glykolethergruppen im Molekül, Sulfobernsteinsäuremono- und - dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Unter amphoteren Tensiden versteht der Fachmann die zwitterionische Tenside und ampholytische Tenside.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄- Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugt geeignete ampholytische Tenside sind ausgewählt unter N-Kokosalkylaminopropionat, Kokosacylaminoethylaminopropionat und C₁₂ - C₁₈ - Acylsarcosin.

Es ist erfindungsgemäß besonders bevorzugt, wenn mindestens ein Tensid des Wellmittels ausgewählt wird unter anionischen Tensiden, amphoteren Tensiden, nichtionischen Tensiden.

Ferner ist es wiederum bevorzugt, wenn das Wellmittel mindestens ein nichtionisches Tensid und mindestens ein amphoteres Tensid enthält. Dabei ist es wiederum bevorzugt, wenn das Wellmittel mindestens ein nichtionisches Tensid und mindestens eine Monocarbonsäure mit einem (C₈ bis C₂₄)-Alkylamid0-(C₂ bis C₃)-alkylamino-Rest enthält.

Das Wellmittel enthält die Tenside bevorzugt in einer Menge von 0,5 bis 5,0 Gew.-%, insbesondere in einer Menge von 0,8 bis 3,0 Gew.-%, jeweils bezogen auf das Gewicht des Wellmittels.

Außerdem sind solche erfindungsgemäßen Dauerwellsysteme bevorzugt, bei denen mindestens ein Tensid des Fixiermittels ausgewählt wird unter anionischen Tensiden, amphoteren Tensiden, nichtionischen Tensiden, kationischen Tensiden.

So ist es wiederum bevorzugt, wenn das Fixiermittel mindestens ein nichtionisches Tensid und mindestens ein kationisches Tensid enthält. Dabei ist es wiederum bevorzugt, wenn das Fixiermittel mindestens ein nichtionisches Tensid mit einer Aminoxid-Gruppe und mindestens ein kationisches Tensid enthält.

Das Fixiermittel enthält die Tenside bevorzugt in einer Menge von 0,5 bis 5,0 Gew.-%, insbesondere in einer Menge von 0,8 bis 3,0 Gew.-%, jeweils bezogen auf das Gewicht des Fixiermittels.

Eine besonders bevorzugte Ausführungsform bedeuten Dauerwellsysteme in Form eines "Kit-of-parts", umfassend
(i) mindestens einen mit einer Verschäumvorrichtung ausgestatteten ersten nichtaerosol-Behälter in Form eines Quetschbehälters, der ein flüssiges Wellmittel enthält, welches in einem kosmetischen Träger eine Kombination aus
   - mindestens einer keratinreduzierenden Verbindung,
   - mindestens einem nichtionischen Tensid und
   - mindestens einem amphoteren Tensid umfasst, und
(ii) mindestens einen mit einer Verschäumvorrichtung ausgestatteten zweiten nichtaerosol-Behälter in Form eines Quetschbehälters, der ein flüssiges Fixiermittel enthält, welches in einem kosmetischen Träger eine Kombination aus
   - mindestens einem Oxidationsmittel,
   - mindestens einem nichtionischen Tensid und
   - mindestens einem kationischen Tensid umfasst.

Eine ganz besonders bevorzugte Ausführungsform sind Dauerwellsysteme in Form eines "Kit-of-parts", umfassend
(i) mindestens einen mit einer Verschäumvorrichtung ausgestatteten ersten nichtaerosol-Behälter in Form eines Quetschbehälters, der ein flüssiges Wellmittel enthält, welches in einem kosmetischen Träger eine Kombination aus
   - mindestens einer keratinreduzierenden Verbindung mit mindestens einer Thiolgruppe,
   - mindestens einem nichtionischen Tensid und
   - mindestens einem amphoteren Tensid umfasst, und
(ii) mindestens einen mit einer Verschäumvorrichtung ausgestatteten zweiten nichtaerosol-Behälter in Form eines Quetschbehälters, der ein flüssiges Fixiermittel enthält, welches in einem kosmetischen Träger eine Kombination aus
   - Wasserstoffperoxid,
   - mindestens einem nichtionischen Tensid und
   - mindestens einem kationischen Tensid umfasst.

Eine ganz besonders bevorzugte spezielle Ausführungsform sind wiederum Dauerwellsysteme in Form eines "Kit-of-parts", umfassend
(i) mindestens einen mit einer Verschäumvorrichtung ausgestatteten ersten nichtaerosol-Behälter in Form eines Quetschbehälters, der ein flüssiges Wellmittel enthält, welches in einem kosmetischen Träger eine Kombination aus
   - mindestens einer keratinreduzierenden Verbindung mit mindestens einer Thiolgruppe,
   - mindestens einem nichtionischen Tensid und
   - mindestens einem amphoteren Tensid in Form einer Monocarbonsäure mit einem (C₈ bis C₂₄)-Alkylamido-(C₂ bis C₃)-alkylamino-Rest umfasst, und
(ii) mindestens einen mit einer Verschäumvorrichtung ausgestatteten zweiten nichtaerosol-Behälter in Form eines Quetschbehälters, der ein flüssiges Fixiermittel enthält, welches in einem kosmetischen Träger eine Kombination aus
   - Wasserstoffperoxid,
   - mindestens einem nichtionischen Tensid mit einer Aminoxid-Gruppe und
   - mindestens einem kationischen Tensid umfasst.

Weiterhin können die Wellmittel wellkraftverstärkende Komponenten enthalten, wie beispielsweise
- heterocyclische Verbindungen wie Imidazol, Pyrrolidin, Piperidin, Dioxolan, Dioxan, Morpholin und Piperazin sowie Derivate dieser Verbindungen wie beispielsweise die C₁₋₄-Alkyl-Derivate, C₁₋₄-Hydroxyalkyl-Derivate und C₁₋₄-Aminoalkyl-Derivate. Bevorzugte Substituenten, die sowohl an Kohlenstoffatomen als auch an Stickstoffatomen der heterocyclischen Ringsysteme positioniert sein können, sind Methyl-, Ethyl-, β-Hydroxyethyl- und β-Aminoethyl-Gruppen. Erfindungsgemäß bevorzugte Derivate heterocyclischer Verbindungen sind beispielsweise 1-Methylimidazol, 2-Methylimidazol, 4(5)-Methylimidazol, 1,2-Dimethylimidazol, 2-Ethylimidazol, 2-Isopropylimidazol, N-Methylpyrrolidon, 1-Methylpiperidin, 4-Methylpiperidin, 2-Ethylpiperidin, 4-Methylmorpholin, 4-(2-Hydroxyethyl)morpholin, 1-Ethylpiperazin, 1-(2-Hydroxyethyl)piperazin, 1-(2-Aminoethyl)piperazin. Weiterhin erfindungsgemäß bevorzugte Imidazolderivate sind Biotin, Hydantoin und Benzimidazol. Ganz besonders bevorzugt ist das Imidazol.
- Aminosäuren wie insbesondere Arginin, Citrullin, Histidin, Ornithin und Lysin. Die Aminosäuren können sowohl als freie Aminosäure als auch als Salze, z. B. als Hydrochloride, eingesetzt werden. Weiterhin haben sich auch Oligopeptide aus durchschnittlich 2-3 Aminosäuren, die einen hohen Anteil (> 50 %, insbesondere > 70 %) an den genannten Aminosäuren haben, als erfindungsgemäß einsetzbar erwiesen. Erfindungsgemäß besonders bevorzugt sind Arginin sowie dessen Salze und argininreiche Oligopeptide.
- Diole wie beispielsweise 2-Ethyl-1,3-hexandiol, 1,3-Butandiol, 1,4-Butandiol, 1,2-Propandiol, 1,3-Propandiol, Neopentylglykol und Ethylenglykol. 1,3-Diole, insbesondere 2-Ethyl-1,3-hexandiol und 1,3-Butandiol, haben sich als besonders gut geeignet erwiesen.

Die wellkraftverstärkenden Verbindungen können in den erfindungsgemäßen Mitteln in Mengen von 0,5 bis 5 Gew.-%, bezogen auf das gesamte Mittel, enthalten sein. Mengen von 1 bis 4 Gew.-%, im Falle der Diole von 0,5 bis 3 Gew.-%, haben sich als ausreichend erwiesen, weshalb diese Mengen besonders bevorzugt sind.

Darüberhinaus kann das Wellmittel zusätzlich weitere Komponenten enthalten, die die Wirkung der keratinreduzierenden Verbindung auf das Keratin fördern. Solche Komponenten sind z.B. Quellmittel für keratinhaltige Fasern wie z.B. C₁-C₆-Alkohole und wasserlösliche Glykole oder Polyole wie z.B. Glycerin, 1,2-Propylenglykol oder Sorbit und Harnstoff oder Harnstoffderivate wie z.B. Allantoin und Guanidin sowie Imidazol und dessen Derivate. Eine bevorzugte weitere Komponente ist 1,2-Propylenglykol, insbesondere in einer Menge von 0,1 bis 5 Gew.-%. Die Mengenangaben beziehen sich jeweils auf die gesamte wässrige Zusammensetzung. In einer bevorzugten Ausführung enthält die wässrige Zusammensetzung 0 bis 5 Gew.-% 1,2-Propylenglykol und/oder 0 bis 5 Gew.-% Harnstoff.

Sowohl das Wellmittel als auch das Fixiermittel können zusätzlich Silikone enthalten. Erfindungsgemäß verwendbare Silikone sind bevorzugt lineare, cyclische oder verzweigte Silikone, ausgewählt aus den Typen der Cyclomethicone, Dimethiconole, Dimethiconcopolyole, Amodimethicone, Trimethylsilylamodimethicone und Phenyltrimethicone. Diese Silikontypen sind dem Fachmann unter der Nomenklatur der Cosmetic, Toiletry and Fragrance Association (CTFA) bekannt und in: M.D. Berthiaume, *Society of the Cosmetic Chemists Monograph Series*, "Silicones in Hair Care", Ed.: L. D. Rhein, Hrsg.: Society of the Cosmetic Chemists, 1997, Kapitel 2 offenbart, worauf an dieser Stelle explizit verwiesen wird. Polysiloxane, wie Dialkyl- und Alkylarylsiloxane, beispielsweise Dimethylpolysiloxan und Methylphenylpolysiloxan, sowie deren alkoxylierte Analoga, mit Hydroxylgruppen terminierte Analoga und quaternierte Analoga, sowie cyclische Siloxane. Dabei sind die Silikone mit den INCI-Bezeichnungen Dimethicone, PEG-12 Dimethicone, PEG/PPG-18/18 Dimethicone, Cyclomethicone, Dimethiconol, Quaternium-80 und Amodimethicone sowie deren Gemische besonders bevorzugt.

Beispiele für solche Silikone sind die von Dow Corning unter den Bezeichnungen DC 190 (INCI-Bezeichnung: PEG/PPG-18/18 Dimethicone), DC 193 (INCI-Bezeichnung: PEG-12 Dimethicone), DC 200, DC1401 (INCI-Bezeichnung: Cyclomethicone, Dimethiconol) und DC 1403 (INCI-Bezeichnung: Dimethicone, Dimethiconol) vertriebenen Produkte sowie die Handelsprodukte DC 244 (INCI-Bezeichnung: Cyclomethicone), DC 344 (INCI-Bezeichnung: Cyclomethicone) und DC 345 (INCI-Bezeichnung: Cyclomethicone) von Dow Corning, Q2-7224 (Hersteller: Dow Corning; ein stabilisiertes Trimethylsilylamodimethicon, Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil Quat 3270 und 3272 (Hersteller: Th. Goldschmidt; diquaternäre Polydimethylsiloxane, INCI-Bezeichnung: Quaternium-80).

Die Silikone sind bevorzugt in den Mengen von 0,1 bis 10 Gew.-%, besonders bevorzugt von 0,3 bis 5 Gew.-%, jeweils bezogen auf das Gewicht des entsprechenden Mittels enthalten.

Das Wellmittel oder das Fixiermittel, insbesondere das Wellmittel, kann zusätzlich Proteinhydrolysate enthalten. Proteinhydrolysate sind Produktgemische, die durch sauer, basisch oder enzymatisch katalysierten Abbau von Proteinen (Eiweißen) erhalten werden. Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (RITA Corp.), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben. Ein bevorzugtes Proteinhydrolysat ist das Seidenproteinhydrolysat (Promois^{®} Silk 720, Promois^{®} Silk 1000).

Erfindungsgemäß ist ebenso die Verwendung von Proteinhydrolysaten pflanzlichen Ursprungs möglich, z. B. Soja-, Mandel-, Reis-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.

Erfindungsgemäß besonders bevorzugt sind Arginin, Asparagin, Asparaginsäure sowie deren Salze und Oligopeptide bzw. Hydrolysate, welche reich an den genannten bevorzugten Aminosäuren sind. Ganz besonders bevorzugt sind Asparagin und Asparaginsäure sowie deren Salze bzw. Hydrolysate.

Sowohl das Wellmittel als auch das Fixiermittel liegen bevorzugt als wässrige Zusammensetzung vor. Eine wäßrige Zusammensetzung im Sinne der Erfindung enthält mindestens 50 Gew.% Wasser bezogen auf das Gewicht der gesamten Zusammensetzung. Diese wäßrige Zusammensetzung kann in verschiedenen Formen, beispielsweise als Lotion, Öl-in-Wasser-Emulsion oder Wasser-in-Öl-Emulsion, vorliegen.

Bevorzugt enthält das Wellmittel und/oder das Fixiermittel weiterhin mindestens einen konditionierenden Wirkstoff.

Als konditionierende Wirkstoffe kommen bevorzugt kationische Polymere in Betracht. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate.
- Polysiloxane mit quaternären Gruppen,
- Polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere.
- Copolymere des Vinylpyrrolidons mit quaternierten Derivaten des Dialkylaminoacrylats und - methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich.
- Vinylpyrrolidon-Vinylimidazoliniummethochlorid-Copolymere, wie sie unter der Bezeichnung Luviquat^{®} angeboten werden,
- quaternierter Polyvinylalkohol
sowie die unter den Bezeichnungen
- Polyquaternium 2,
- Polyquaternium 17,
- Polyquaternium 18 und
- Polyquaternium 27
bekannten Polymeren mit quartären Stickstoffatomen in der Polymerhauptkette.

Geeignet als konditionierende Wirkstoffe sind auch Ampho-Polymere. Unter dem Oberbegriff Ampho-Polymere sind amphotere Polymere, d. h. Polymere, die im Molekül sowohl freie Aminogruppen als auch freie -COOH- oder SO₃H-Gruppen enthalten und zur Ausbildung innerer Salze befähigt sind, zwitterionische Polymere, die im Molekül quartäre Ammoniumgruppen und - COO⁻- oder -SO₃⁻-Gruppen enthalten, und solche Polymere zusammengefaßt, die -COOH- oder SO₃H-Gruppen und quartäre Ammoniumgruppen enthalten. Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt. Ebenfalls bevorzugte Amphopolymere setzen sich aus ungesättigten Carbonsäuren (z. B. Acryl- und Methacryl-säure), kationisch derivatisierten ungesättigten Carbonsäuren (z. B. Acrylamidopropyl-trimethyl-ammoniumchlorid) und gegebenenfalls weiteren ionischen oder nichtionogenen Monomeren zusammen, wie beispielsweise in der deutschen Offenlegungsschrift 39 29 973 und dem dort zitierten Stand der Technik zu entnehmen sind. Terpolymere von Acrylsäure, Methylacrylat und Methacrylamidopropyltrimoniumchlorid, wie sie unter der Bezeichnung Merquat^{®}2001 N im Handel erhältlich sind sowie das Handelsprodukt Merquat^{®} 280, sind erfindungsgemäß besonders bevorzugte Ampho-Polymere.

Die kationischen oder amphoteren Polymere sind in den erfindungsgemäßen Zubereitungen bevorzugt in Mengen von 0,1 bis 5 Gew.-%, bezogen auf die gesamte Zubereitung, enthalten.

Alle Inhaltsstoffe des flüssigen Wellmittels bzw. des flüssigen Fixiermittels sind in einen kosmetischen Träger eingearbeitet. Solche kosmetischen Träger sind physiologisch verträglich. Bevorzugte kosmetische Träger sind wässrige Träger, alkoholische Träger oder wässrigalkoholische Träger mit vorzugsweise mindestens 10 Gew.-% Wasser, bezogen auf das gesamte Mittel. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Bezüglich der weiteren Bestandteile der erfindungsgemäßen Mittel und deren übliche Einsatzmengen wird ausdrücklich auf die bekannten Monographien, z. B. Umbach, Kosmetik, 2. Auflage, Georg Thieme Verlag, Stuttgart New York, 1995, und Kh. Schrader, Grundlagen und Rezepturen der Kosmetika, 2. Auflage, Hüthig Buch Verlag, Heidelberg, 1989, verwiesen.

Ein zweiter Gegenstand der Erfindung ist ein Verfahren zur dauerhaften Umformung keratinhaltiger Fasern, insbesondere menschlicher Haare, in welchem
(i) die Fasern unter Zuhilfenahme von Verformungshilfsmitteln nach, vor oder während des Schritts (ii) verformt werden,
(ii) ein in einem nichtaerosol-Behälter mit Verschäumvorrichtung konfektioniertes, flüssiges Wellmittel, welches in einem kosmetischen Träger eine Kombination mindestens einer keratinreduzierenden Verbindung mit mindestens einem Tensid umfasst, als Nichtaerosolschaum auf die Fasern aufgetragen wird,
(iii) die Fasern nach einer Einwirkzeit Z1, gespült und gegebenenfalls getrocknet werden,
(iv) anschließend ein in einem nichtaerosol-Behälter mit Verschäumvorrichtung konfektioniertes, flüssiges Fixiermittel, welches in einem kosmetischen Träger eine Kombination aus mindestens einem Oxidationsmittel mit mindestens einem Tensid umfasst, auf die Fasern aufgetragen und nach einer Einwirkzeit Z2 wieder abgespült wird.

Verformungshilfsmittel im Sinne des erfindungsgemäßen Verfahrens können
- z.B. Lockenwickler oder Papilloten im Falle einer Dauerwelle,
- oder Hilfsmittel für eine mechanische Glättung, wie ein Kamm oder eine Bürste, ein Glättungsboard oder ein beheizbares Glättungseisen im Falle einer Haarglättung sein.
Wenn die Verformungshilfsmittel, beispielsweise Wickler, im Rahmen eines Dauerwellverfahrens für einen längeren Zeitraum an der Faser befestigt werden, so ist es zweckmäßig, diese Verformungshilfsmittel nach Ablauf der Einwirkzeit Z1 aus Schritt (iii) (vor oder nach dem Spülen) oder nach Schritt (iv) (oder während Schritt (iv) nach Ablauf der Einwirkzeit Z2) zu entfernen. Es kann in diesem Zusammenhang vorteilhaft sein, die Verformungshilfsmittel während des Schritts (iv) im Haar zu belassen, sie danach zu entfernen und danach Schritt (iv) als sogenannten Nachfixierschritt (v) zu wiederholen.

In einer bevorzugten Ausführungsform der Erfindung werden die keratinhaltigen Fasern vor dem Schritt (i) angefeuchtet. Dies kann durch Besprühen der Fasern mit einer Flüssigkeit, bevorzugt mit Wasser, geschehen. Bevorzugterweise werden die Fasern vor Schritt (i) mit einem herkömmlichen Shampoo shampooniert, gespült und dann mit einem Handtuch frottiert. Nach Abschluß des Frottierschritts bleibt eine fühlbare Restfeuchtigkeit im Haar zurück.

Unter einer mechanischen Glättung wird erfindungsgemäß eine Streckung der krausen Faser entlang ihrer längsten räumlichen Ausdehnung verstanden.

Die Einwirkzeit Z1 beträgt bevorzugt 5-60 Minuten, besonders bevorzugt 10-30 Minuten.

Die Einwirkzeit Z2 beträgt bevorzugt 1-30 Minuten, besonders bevorzugt 5-20 Minuten.

Für den zweiten Erfindungsgegenstand gelten die erfindungsgemäß bevorzugten Ausführungsformen des ersten Erfindungsgegenstandes mutatis mutandis.

Die folgenden Beispiele sollen die Erfindung näher erläutern.

### Beispiele

Es wurden das folgende Wellmittel und das folgende Fixiermittel bereitgestellt:
Wellmittel:

| Rohstoff | Menge in Gew.-% |
|---|---|
| Ammoniumthioglykolat (71 Gew.-% in Wasser) | 15,60 |
| Etidronsäure | 0,20 |
| Ammoniak (25 Gew.-%ig in Wasser) | 1,70 |
| Ammoniumbicarbonat | 4,50 |
| Dehyton K ¹ | 2,20 |
| Eumulgin L ² | 0,50 |
| Cremophor CO 60 ³ | 0,80 |
| Parfum | 0,50 |
| Herbasol Extrakt Aloe ⁴ | 0,60 |
| Polyquaternium-6 | 0,10 |
| Gluadin W 20 ⁵ | 0,10 |
| destilliertes Wasser | ad 100 |

| | |
|---|---|
| ¹ N,N-Dimethyl-N-(C₈₋₁₈-kokosamidopropyl)ammoniumaceto-betain (ca. 30% Aktivsubstanz; INCI-Bezeichnung: Aqua (Water), Cocamidopropyl Betaine) (Cognis) ² Laurylglykolether, ethoxyliert mit 1 Einheit Propylenoxid und 9 Einheiten Ethylenoxid (INCI-Bezeichnung: PPG-1-PEG-9 Lauryl Glycol Ether) (Cognis) ³ hydriertes Rizinusöl mit ca. 60 EO-Einheiten (INCI-Bezeichnung: PEG-60 Hydrogenated Castor Oil) (BASF SE) ⁴ Aloe Vera-Extrakt (3 Gew.-% Aktivsubstanz; INCI-Bezeichnung: Aqua (Water), Propylene Glycol, Aloe Barbadensis Leaf Extract, Sorbitol ,Phenoxyethanol, Methylparaben, Ethylparaben, Propylparaben, Butylparaben, Isobutylparaben) (Cosmetochem) ⁵ Weizenproteinhydrolysat (mind. 20% Festkörper; INCI-Bezeichnung: Aqua (Water), Hydrolyzed Wheat Protein, Sodium Benzoate, Phenoxyethanol, Methylparaben, Propylparaben) (Cognis) | |

Fixiermittel:

| Rohstoff | Menge in Gew.-% |
|---|---|
| Phosphorsäure (85 Gew.-%ig in Wasser) | 0,95 |
| Wasserstoffperoxid | 2,00 |
| Methylparaben | 0,04 |
| Dehyquart A CA ⁶ | 0,30 |
| Polyquaternium-6 | 0,50 |
| Aromox MCD W ⁷ | 3,00 |
| Parfum | 0,30 |
| destilliertes Wasser | ad 100 |

| | |
|---|---|
| ⁶ Trimethylhexadecylammoniumchlorid (ca. 24 -26 % Aktivsubstanz; INCI-Bezeichnung: Aqua (Water), Cetrimonium Chloride) (Cognis) ⁷ N,N-Dimethyl-N-kokosalkylamin-N-oxid (ca. 30% Aktivsubstanz; INCI-Bezeichnung: Cocamine Oxide) (Akzo Nobel) | |

Das Wellmittel und das Fixiermittel wurden je in einen Quetschbehälter mit Verschäumvorrichtung (Squeeze Foamer der Fa. Daiwa Can Company) gefüllt. Die in dieser Form abgefüllten Mittel wurden einem Probanden zur Selbstapplikation im Rahmen einer Dauerwellanwendung ausgehändigt. Der Proband führte selbstständig eine Dauerwellanwendung aus, in dem er handtuchtrockenes Haar auf Wickler wickelte, das Wellmittel als Nichaerosolschaum nahezu tropffrei auftrug, nach einer Einwirkzeit von 25 Minuten das Haar spülte und anschließend das Fixiermittel als Nichtaerosolschaum nahezu tropffrei auftrug. Nach einer Einwirkzeit von 20 Minuten entfernte der Proband die Wickler, spülte das Haar und trocknete es.

Es wurde unter Selbstanwendung eine gute Haarumformung erzielt.

## Patentansprüche

1. Kit-of-parts, umfassend
(i) mindestens einen mit einer Verschäumvorrichtung ausgestatteten ersten nichtaerosol-Behälter, der ein flüssiges Wellmittel enthält, welches in einem kosmetischen Träger eine Kombination mindestens einer keratinreduzierenden Verbindung mit mindestens einem Tensid umfasst, und
(ii) mindestens einen mit einer Verschäumvorrichtung ausgestatteten zweiten nichtaerosol-Behälter, der ein flüssiges Fixiermittel enthält, welches in einem kosmetischen Träger eine Kombination aus mindestens einem Oxidationsmittel mit mindestens einem Tensid umfasst.

2. Kit-of-parts nach Anspruch 1, **dadurch gekennzeichnet, dass** der erste nichtaerosol-Behälter und der zweite nichtaerosol-Behälter als Quetschbehälter ausgestaltet sind.

3. Kit-of-parts nach einem der Ansprüche 1 oder 2, **dadurch gekennzeichnet, dass** mindestens eine keratinreduzierende Verbindung des flüssigen Wellmittels ausgewählt wird unter Cysteamin, Cystein, Bunte Salzen und Salzen der schwefligen Säure, Thioglykolsäure, Thiomilchsäure, Thioäpfelsäure, Mercaptoethansulfonsäure sowie den Salzen und Estern der vorgenannten Thiosäuren.

4. Kit-of-parts nach einem der Ansprüche 1 bis 3, **dadurch gekennzeichnet, dass** das Wellmittel die keratinreduzierende Verbindung in einer Menge von 5,0 bis 20,0 Gew.-% bezogen auf das Gewicht des Wellmittels enthält.

5. Kit-of-parts nach einem der Ansprüche 1 bis 4, **dadurch gekennzeichnet, dass** mindestens ein Tensid des Wellmittels ausgewählt wird unter anionischen Tensiden, amphoteren Tensiden, nichtionischen Tensiden.

6. Kit-of-parts nach einem der Ansprüche 1 bis 5, **dadurch gekennzeichnet, dass** Wellmittel mindestens ein nichtionisches Tensid und mindestens ein amphoteres Tensid enthält.

7. Kit-of-parts nach einem der Ansprüche 1 bis 6, **dadurch gekennzeichnet, dass** das Wellmittel die Tenside in einer Menge von 0,5 bis 5,0 Gew.-%, insbesondere in einer Menge von 0,8 bis 3,0 Gew.-%, jeweils bezogen auf das Gewicht des Wellmittels, enthält.

8. Kit-of-parts nach einem der Ansprüche 1 bis 7, **dadurch gekennzeichnet, dass** mindestens ein Tensid des Fixiermittels ausgewählt wird unter anionischen Tensiden, amphoteren Tensiden, nichtionischen Tensiden, kationischen Tensiden.

9. Kit-of-parts nach einem der Ansprüche 1 bis 8, **dadurch gekennzeichnet, dass** das Fixiermittel mindestens ein nichtionisches Tensid und mindestens ein kationisches Tensid enthält.

10. Kit-of-parts nach einem der Ansprüche 1 bis 9, **dadurch gekennzeichnet, dass** das Fixiermittel die Tenside in einer Menge von 0,5 bis 5,0 Gew.-%, insbesondere in einer Menge von 0,8 bis 3,0 Gew.-%, jeweils bezogen auf das Gewicht des Fixiermittels, enthält.

11. Mittel nach einem der Ansprüche 1 bis 10, **dadurch gekennzeichnet, dass** die Verschäumvorrichtung eine Mischkammer, mindestens eine Schaumerzeugungseinrichtung in Form eines Netzes und einen Schaumkanal umfasst.

12. Mittel nach Anspruch 11, **dadurch gekennzeichnet, dass** das Netz eine Lochdichte von 50 bis 220 mesh aufweist.

13. Verfahren zur dauerhaften Umformung keratinhaltiger Fasern, insbesondere menschlicher Haare, in welchem
(i) die Fasern unter Zuhilfenahme von Verformungshilfsmitteln nach, vor oder während des Schritts (ii) verformt werden,
(ii) ein in einem nichtaerosol-Behälter mit Verschäumvorrichtung konfektioniertes, flüssiges Wellmittel, welches in einem kosmetischen Träger eine Kombination mindestens einer keratinreduzierenden Verbindung mit mindestens einem Tensid umfasst, als Nichtaerosolschaum auf die Fasern aufgetragen wird,
(iii) die Fasern nach einer Einwirkzeit Z1, gespült und gegebenenfalls getrocknet werden,
(iv) anschließend ein in einem nichtaerosol-Behälter mit Verschäumvorrichtung konfektioniertes, flüssiges Fixiermittel, welches in einem kosmetischen Träger eine Kombination aus mindestens einem Oxidationsmittel mit mindestens einem Tensid umfasst, auf die Fasern aufgetragen und nach einer Einwirkzeit Z2 wieder abgespült wird.
